# EUROPEAN PATENT SPECIFICATION

(11) **EP 4 312 852 B1**
(45) Date of publication and mention of the grant of the patent: **21.10.2026**
(21) Application number: 22716409.2
(22) Date of filing: 22.03.2022
(51) Int. Cl.: A61B 34/10

(54) **SYSTEM FOR LOCATING OBJECT(S) IN PARTICULAR FOR OPTIMAL LOWER LIMB SURGERY PLANNING USING X-RAY IMAGES**
SYSTEM ZUR ORTUNG VON OBJEKT(EN), INSBESONDERE FÜR OPTIMALE UNTERSCHENKELCHIRURGIEPLANUNG MITHILFE VON RÖNTGENBILDERN
SYSTÈME DE LOCALISATION D'OBJET(S), EN PARTICULIER POUR LA PLANIFICATION CHIRURGICALE OPTIMALE DES MEMBRES INFÉRIEURS À L'AIDE D'IMAGES RADIOGRAPHIQUES

(30) Priority: 23.03.2021 EP 21305355
(43) Date of publication of application: 07.02.2024
(73) Proprietor: Kyniska Robotics, 38400 Saint-Martin-d'Hères (FR)
(72) Inventor: CHANRION, Marie-Anne, 38400 SAINT MARTIN D'HERES (FR); DELMON, Vivien, 38400 SAINT MARTIN D'HERES (FR); FONTAINE, Sylvain, 38400 SAINT-MARTIN-D'HÈRES (FR)
(74) Representative: Regimbeau
(86) International application number: PCT/EP2022/057414
(87) International publication number: WO 2022/200306

(56) References cited:
- WO-A1-2013/127735
- WO-A1-2018/236936
- US-A1- 2018 256 259
- US-B2- 8 948 471

## Description

### FIELD OF THE INVENTION

The present invention relates to a system for locating object(s) in several regions of interest of a patient including a hip joint, a knee joint, an ankle joint and for measuring alignment parameters including a femur bone and a tibia bone in particular for planning a surgery involving the alignment of two bones and in particular for the surgery around a knee joint, knee arthroplasty, tibial osteotomy or femoral osteotomy, or any complex osteotomy surgery on the femur or tibia.

### BACKGROUND OF THE INVENTION

To relieve pain, many surgeries of the lower limb (including the joints) require the diagnosis of the leg bones alignment. The surgical procedure will then aim, among other specific goals to the pathology, to re-align the limb. The correction needed for treating the pathological alignment is established by the surgeon based on the pathology. Under or over correction may jeopardize the surgical procedure.

Hence, establishing the precise alignment parameters of the pathological limb is the core of the diagnosis and further execution of surgical corrective procedure.

To establish the diagnosis of the limb alignment, it is of common practice to access the limb alignment in 2D using the long leg radiograph or in 3D using a CT scan pre-operatively which have the disadvantages of being an irradiating procedure. Such pre-operative imaging requires registration of the patient in the operating room after. Also, in case these images have to be used in per-operative, further calibration of these images with surgical tools or further images acquired during the surgery has to be made.

Furthermore, it is well known that errors in the alignment can be introduced if the leg is not consistently positioned preoperatively, intraoperatively and postoperatively.

Thus, with these methods, the planning of the surgical procedure and its implementation in the operating room is lengthy and may be inaccurate causing pain and/or reduced quality of life on the patient.

US2018256259A1 discloses a method which involves obtaining a target image of a bone of a patient. A transformation matrix that transforms a contour of a portion of the bone is generated in an initial image to satisfy a defined rule for conforming to a contour of a corresponding portion of the bone in the target image. A transformed location data structure is generated based on applying the transformation matrix to the initial location data structure. Graphical representation of the surgical implant device overlaid at locations on the target image of the bone determined based on the transformed location data structure is displayed.

WO2018236936A1 discloses a method of tracking motion of a body part, the method comprising: (a) gathering motion data from a body part repositioned within a range of motion, the body part having mounted thereto a motion sensor; (b) gathering a plurality of radiographic images taken of the body part while the body part is in different positions within the range of motion, the plurality of radiographic images having the body part and the motion sensor within a field of view; and, (c) constructing a virtual three dimensional model of the body part from the plurality of radiographic images using a structure of the motion sensor identifiable within at least two of the plurality of radiographic images to calibrate the radiographic images.

### SUMMARY OF THE INVENTION

The invention permits to solve at least the above-mentioned drawbacks.

The invention is defined in the appended claims.

The invention permits to locate in the operating room, the characteristic points of the limb, in particular, in order to measure in the operating room, alignment parameters of the limb.

The invention permits to follow, in real time, limb alignment parameters, based on the patient specific limb images to execute an optimal 3D surgery.

This method, not forming part of the present invention, should be automated and conducted intra-operatively in a very short amount of time relatively to the surgery time.

This is done by means of trackers fixed to the femur bone and the tibia bone, where each tracker defines its referential: Tracker T_F, reference system Ref_F for the femur and Tracker T_T, reference system Ref_T for the tibia.

The invention allows to plan optimal femoral cuts to position a total knee femur prosthesis size, position, and orientation in the reference system of the tracker fixed to the femur and/or an optimal tibial cut to position a total knee tibia prosthesis size, position, and orientation in the reference system of the tracker fixed to the tibia bone, wherein said optimal cuts are performed using robotics or surgical navigation assistance.

The invention allows to plan optimal femoral cuts to position a uni-compartimental knee femur prosthesis size, position, and orientation in the reference system of the tracker fixed to the femur and/or optimal tibial cuts to position a uni-compartimental knee tibia prosthesis size, position, and orientation in the reference system of the tracker fixed to the tibia bone, wherein said optimal cuts are performed using robotics or surgical navigation assistance.

The invention allows to plan optimal femoral cuts to position a specific partial knee or cruciate retaining TKA or bicruciate retaining TKA femur prosthesis size, position, and orientation in the reference system of the tracker fixed to the femur and/or optimal tibial cuts to position a specific partial knee or cruciate retaining TKA or bicruciate retaining TKA tibia prosthesis size, position, and orientation in the reference system of the tracker fixed to the tibia bone, wherein said optimal cuts are performed using robotics or surgical navigation assistance.

### BRIEF DESCRIPTION OF THE DRAWINGS

Other features and advantages of the invention will appear in the following description. Embodiments of the invention will be described with reference to the drawings, in which:
- figure 1 illustrates bone structure of a lower limb;
- figure 2 illustrates systems in which the invention belongs to;
- figure 3 illustrates the steps of a method not forming part of the present invention;
- figures 4 to 6 illustrate regions of interest involved in the invention;
- figure 7 illustrates the localization of trackers during an osteotomy of the lower limb.

On the figures, similar elements are identified with the same reference number.

### DETAILED DESCRIPTION OF THE INVENTION

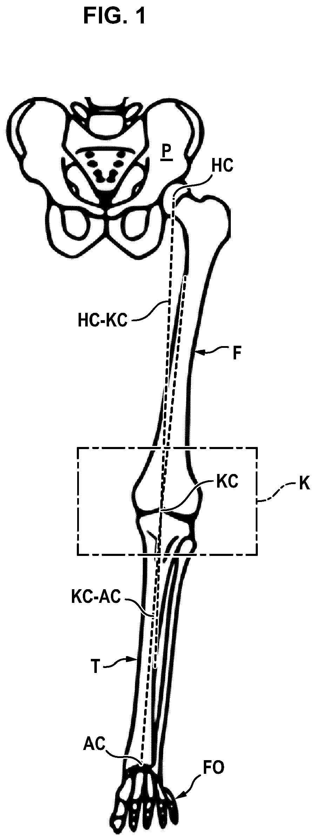

**Figure 1** shows a lower limb comprising a tibia bone T and a femur bone F. Both bones T and F are articulated around a knee K called knee joint. The knee joint has a knee center KC. There are commonly multiple definitions of the knee center. It can be defined, for instance, as the center of the apex of the femoral notch, the midpoint of the femoral condyles, the center of tibial spines, the midpoint of soft tissue outline or the midpoint of tibial plateaus.

The femur bone F has a proximal joint, the hip joint and a center HC located in the hip ball (hereafter hip center HC) which makes the interface with the pelvis P. The tibia bone T has a distal joint, the ankle joint and a center AC (here after ankle center AC) which makes the interface with the foot FO. As for the knee center, there are commonly multiple definitions of the AC. The AC can be defined, for instance, as the center of soft tissue near both the malleoli, the center bones near both the malleoli or the center of talus.

The invention is thus applicable to each of the above definitions.

The mechanical axis HC-KC of the femur bone is the line passing through the hip center HC and the knee center KC. The mechanical axis KC-AC of the tibia bone T is the line passing through the knee center KC and the ankle center AC.

During a knee joint surgery, it is necessary to determine parameters in region comprising a joint. In particular, alignment parameters are calculated from at least two characteristic points localized on the surface of the femur and/or the tibia (it can also be called "bony landmarks"). Alternatively, the alignment parameters are calculated from characteristic element(s) which locations are deduced from the locations of two or more characteristic points. For instance, alignment parameters characterizing the above mechanical axis of the tibia and the femur based on the localization of joint centers can be necessary to plan a surgery. To this end, a medical system and an imaging device are involved.

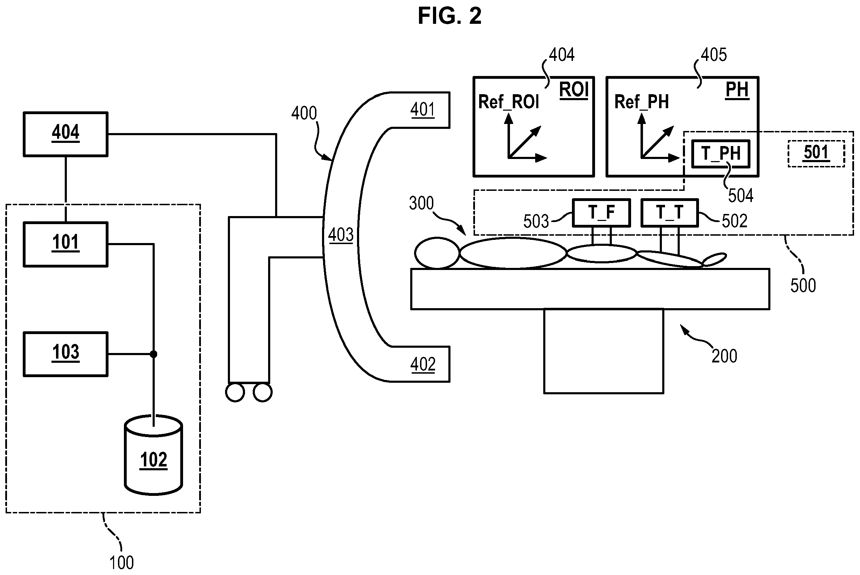

**Figure 2** shows a medical system 100, an operating table 200 on which a patient 300 may be disposed. In particular, the patient 300 lies on this table for surgical lower limb surgical procedure.

The medical system 100 is linked or integrated with an imaging device 400 for acquiring images. The imaging device 400 comprises a detector 401 and an X-ray source 402. The X-ray source 402 and the detector 401 are, in particular, mounted facing each other. This device 400 may be a cone beam computed tomography (CBCT) or a computed tomography (CT) imaging system both well known in the art. The detector 401 and the X-ray source 402 may be arranged at ends of a C-arm 403 which is preferably rotatably mounted around the operating table 200 for acquiring images according to different positions of the C-arm relative to the patient 300.

The imaging device 400 comprises a first processing unit 404 (such as a computer) for acquiring 2D images, reconstructing 3D images from acquired 2D images, and sending them to a second processing unit 101. To this end, the first processing unit 404 is connected to a second processing unit 101 of the medical system 100 by means of any suitable communication link. Alternatively, the two processing units can be merged in case of the imaging system device 400 belongs to the medical system 100.

The medical system 100 comprises a storage unit 102 configured for storing at least the acquired and reconstructed images, the processing unit 101 is configured for implementing methods for locating object(s) (i.e., characteristic point(s), characteristic element(s)), alignment parameters of a femur bone and/or a tibia bone and/or a method for planning a surgery (see hereafter). Furthermore, the system 100 comprises one or several display(s) 103 for the display of images or other parameters that the practitioner needs.

In this description a display is defined as being any suitable device for displaying information to the practitioner or means suitable for augmented reality or virtual reality or computer screen or means for projecting images, etc. In any case, the invention cannot be limited to a particular display.

The imaging system 400 is associated with a navigation system 500 which is a system that includes trackers which position can be known relatively to at least another tracker either with direct mutual detection capabilities or indirectly via, for instance an external localizer 501 with detection capabilities (e.g., an infra-red camera for optical navigation).

The navigation system 500 can be based on:
- mechanics;
- ultrasonics;
- optics;
- electromagnetic;
- a combination of different technologies.

The navigation system can be configured to detect several trackers, alternatively a tracker can be a localizer itself. The tracker can comprise radio detectable elements or not. In any case, we consider that the navigation system 500 is well adapted to detect the trackers.

The method, not forming part of the present invention, for locating characteristic point(s), alignment parameter(s) and the method, not forming part of the present invention, for planning a surgery require a navigation system including one or several trackers rigidly fixed to a bony structure.

A reference system Ref_ROI is defined to an image of a region of interest, it is the reference system in which the images are acquired via the imaging device 400. Any point of an image can be thus located in the reference system Ref_ROI.

A tracker T_PH 504 is fixed to a calibration phantom PH which is always in the field of view of the imaging device during acquisitions of the images of a region of interest. This calibration phantom PH comprises radio-detectable elements that are visible in the images. The calibration phantom PH can be thus visible in an image and permit to link the image to the reference system Ref_ROI. The relative position between the radio-detectable elements of PH and the tracker T_PH is known by calibration.

A tracker T_F 503 is fixed to the femur bone F and defines a reference system Ref_F.

A tracker T_T 502 is fixed to the tibia bone T and defines a reference system Ref_T.

Trackers, T_T, T_F and T_PH can be located in relation to each other by means of the navigation system 500.

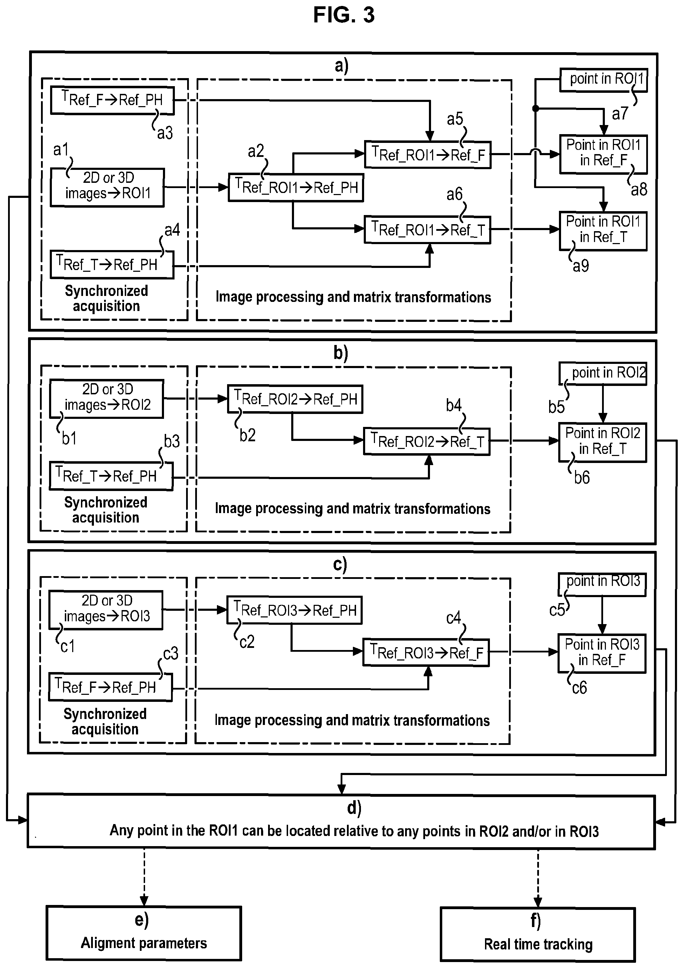

The surgery of the knee, the hip, the ankle requires the localization of at least one characteristic point located in a region comprising the joint. This or these characteristic points are located relatively to the bone belonging to the joint. Therefore, from one or several characteristic points, it is possible to obtain alignment parameter(s) including the femur bone and/or the tibia bone around the knee joint region. These parameters will be defined in accordance with the type of surgery and according to the method described in relation with **figure 3****.**

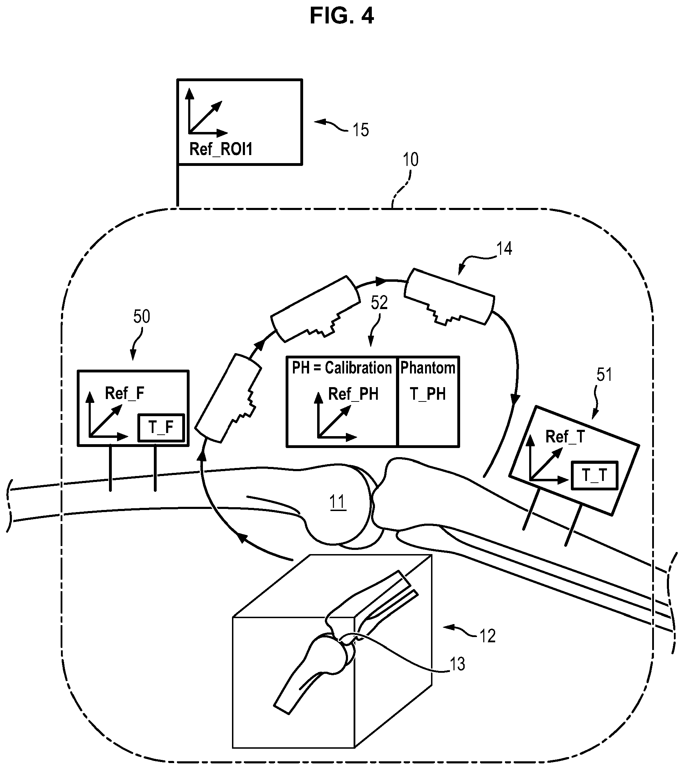

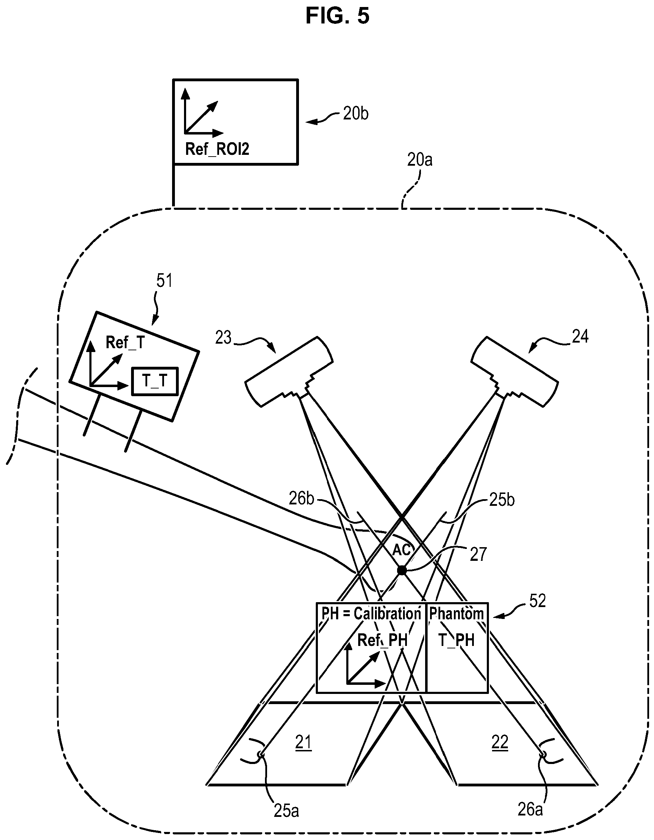

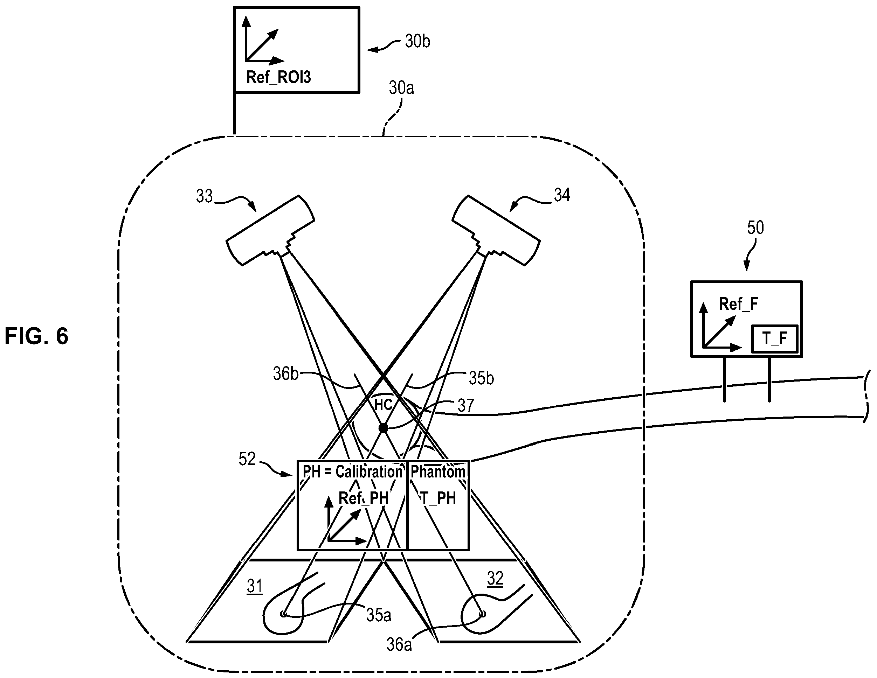

In a lower limb surgery, characteristic points are localized in several independent regions of interest, we thus consider a first region of interest which is the knee joint region (see **figure 4**), a second region of interest which is the ankle joint region (see **figure 5**) and a third region which is the hip joint region (see **figure 6**). Obviously, each region can be considered independently depending on the need for the surgery. Indeed, the locations of any characteristic point in the lower limb being of use for the determination of alignment parameter(s) of a femur bone in relation with a tibia bone in order which are the necessary inputs to plan and execute an optimal surgery around a knee joint of a patient.

In the following, we describe how to locate one or more characteristic point(s) in each region of interest.

### First region of interest: knee joint region

In relation with **figure 4****,** we consider a first region of interest 10 comprising a part of the tibia bone T with the tracker T_T 51 and/or a part of the femur bone F with the tracker T_F 50 and the knee joint 11. Any characteristic point of that first region represented on the image 12 (which can be a 2D or 3D image) has to be located **(****figure 3** step a) relative to the trackers T_T and/or T_F. for instance in **figure 4****,** the characteristic point 13 in the image 12 corresponds to the knee joint 11 in a 3D image 12.

In particular, a transform matrix T_{Ref_ROI1→Ref_F} between the reference system of the first region of interest and the reference system of Ref_F and/or a transform matrix T_{Ref_ROI1→Ref_T} between the reference system of the first region of interest and the reference system of Ref_T are determined.

By means of transform matrix we mean any geometric transformation permitting to switch from a reference system to another reference system. This type of geometric transformation is well known in the art and will not be described further.

One 3D image or at least two 2D images of the first region of interest is acquired (**figure 3** step a1).

As known, a 3D image of the first region of interest is obtained from projections acquired according to different positions 14 of the X-ray source in the reference system Ref_ROI1 15 of the imaging device defined in this first region of interest 10. In particular, the 3D image is obtained by a tomography reconstruction process of the region. The obtention of the 3D image is well known by the man skilled in the art and will not be described further.

In a preferred embodiment, a calibration phantom PH 52 is in the field of view of the imaging device and its radio-detectable elements are visible in the 3D or 2D image. The calibration phantom PH is thus visible in the 3D or 2D image of the first region of interest (**figure 3** step a3, step a4).

Image processing algorithms allow to detect radio-detectable elements of PH in the images and since the relative position between the radio-detectable elements of PH and the tracker T_PH is known by calibration, the transform matrix T_{Ref_ROI1→Ref_PH} between Ref_ROI1 and Ref_PH is determined (**figure 3** step a2).

At the time of the acquisition, the position of the trackers T_T and/or T_F with respect to T_PH are known, matrix transformation T_{Ref_F→Ref_PH} between Ref_F and Ref_PH and/or matrix transformation T_{Ref_T→Ref_PH} between Ref_T and Ref_PH are determined (**figure 3** steps a3 and a4).

For the femur side, transform matrix T_{Ref_ROI1→Ref_F} between Ref_ROI and Ref_F is determined (**figure 3** step a5) from T_{Ref_F→Ref_PH} and T_{Ref_ROI1→Ref_PH}.

For the tibia side, transform matrix T_{Ref_ROI1→Ref_T} between Ref_ROI and Ref_T is determined (**figure 3** step a6) from T_{Ref_T→Ref_PH} and T_{Ref_ROI1→Ref_PH}.

Thus, any characteristic point located in the first region of interest ROI1 (step a7) can be then located with respect to the reference system Ref_F (**figure 3** step a8) and/or Ref_T (**figure 3** step a9) by the use of the matrices determined at steps a5and/or a6).

For instance, the knee joint center can be located in the first region of interest ROI1, via either the analysis of one or more characteristic point found on the images. Precisely, different characteristic points of the image 12 can be used to define the knee center. For instance, it can be the average of two or more points: the midpoint of the femoral condyles of the center of the soft tissue outline.

Alternatively, the knee joint center can be located in the first region of interest ROI1 by using a morpho-functional method using flexion-extension movements. In the morpho-functional method, 3D positions and orientations of the femur and tibia trackers are recorded during the motion by the navigation system. An average rotation axis representing the flexion/extension is estimated. The knee center 13 in image 12 obtained via a morpho-functional method can be adjusted via the information of the position of other characteristic points.

### Second region of interest: ankle joint region (step b)

In relation with **figure 5****,** we consider a second region of interest 20a, comprising a part of the tibia bone T with the tracker T_T 51 and the ankle joint (comprising the ankle center 27) in which the position of any characteristic point of an image (2D or 3D), of the second region of interest has to be located (**figure 3** step b), for instance the ankle center 27 relative to the tracker T_T.

At least two 2D images or one 3D image is acquired (**figure 3** step b1) according to a position of the X-ray source.

In a preferred embodiment where the ankle center has to be located geometrically, at least two projections images 21, 22 of the second region of interest are acquired according to two respective positions 24, 23 of the X-ray source. As illustrated on figure 5, the ankle center is represented on two 2D images 21, 22 by 25a and 25b.

The ankle center AC 25a, 26a as well as any characteristic point of the ankle joint, is visible on the acquired images 21, 22. The calibration phantom PH 52 is also visible on the images.

Image processing algorithms allow to detect radio-detectable elements of PH on the images and since the relative position between the radio-detectable elements of PH and the tracker T_PH is known by calibration, the transform matrix T_{Ref_ROI2→Ref_PH} between Ref_ROI2 and Ref_PH is determined (**figure 3** step b2). To this end, a single 2D image is necessary for the transform matrix T_{Ref_ROI2→Ref_PH}, any supplementary image will improve the redundancy and hence the accuracy of the transform matrix. One can note that in case of a 3D image, it will be the combination of for instance 180 images via a reconstruction algorithm.

At the time of the acquisition, the position of the tracker T_T with respect to T_PH is known, matrix transformation T_{Ref_T→Ref_PH} between Ref_T and Ref_PH is determined (Figure 3 step b3).

Transform matrix T_{Ref_ROI2→Ref_T} between Ref_ROI2 and Ref_T is determined (Figure 3 step b4) from T_{Ref_T→Ref_PH} and T_{Ref_ROI2→Ref_PH}.

Finally, any characteristic point located in the second region of interest ROI in Ref_ROI2 can be located with respect to Ref_T (**figure 3** steps b5 and b6).

In a preferred embodiment, the position (**figure 3** step b5) of any characteristic point in ROI2 is determined from the at least two projection images. To that end, from the positions of this characteristic point in each image 21, 22, corresponding backprojection lines 25b, 26b are calculated, the position of the characteristic point in the reference system of the second region of interest ROI2 being the intersection (or the closest point between the two backprojection lines) of these backprojection lines 25b, 26b.

As known, for each pixel of a 2D image, a backprojection line, is the line between the corresponding pixel in the detector and the X-ray source.

In an embodiment, the ankle center has to located in the second of region of interest. Therefore, for obtaining (**figure 3** step b5) the position of ankle center in the second region of interest, 2D projection images can be orthogonal acquisitions frontal and sagittal to detect characteristic points on the talus and the distal tibia. In particular, the 2D projection images correspond to preferably +15° and - 15° from the frontal orientation to detect malleoli of the tibia and fibula. From these characteristic points, the position of the ankle center is obtained. In that case, the ankle center is a characteristic element, i.e., obtained from at least two characteristic points.

In an alternative embodiment, for obtaining (**figure 3** step b5) the ankle center is to point it (or to point any characteristic point in ROI2) with an external tool (e.g., a tracked probe) that is localized in the ROI2 which means that the position of the ankle center (or any characteristic point in ROI2) will be expressed in the reference system of the second region of interest ROI2 and hence relatively to T_T. In other words, the ankle center can be estimated with a morphologic method. For instance, the tracked probe can point the middle of the medial and lateral point of the ankle. Therefore, no image is needed for locating the characteristic point in the second region of interest.

### Third region of interest: hip joint region (step c)

In relation with figure 6, we consider a third region of interest 30a, comprising a part of the femur bone B with tracker T_F 50 and the hip joint (comprising the hip center 37) in which the position of any characteristic point of an image (2D or 3D), of the third region of interest has to be located (**figure 3** step c)), for instance the hip center 37 relative to the tracker T_F.

At least one 2D image or 3D image is acquired (**figure 3** step c1) according to a position of the X-ray source.

In a preferred embodiment where the hip center has to be located geometrically, at least two projections images 31, 32 of the third region of interest ROI3 are acquired according to two respective positions 34, 33 of the X-ray source. As illustrated on figure 6, the hip center is represented on two 2D images 31, 32 by 35a and 36a.

The hip center HC 35a, 36a, as well as any characteristic point of the hip joint is visible on the acquired image(s) 31, 32. The calibration phantom PH 52 is also visible on the image(s).

Image processing algorithms allow to detect radio-detectable elements of PH on the images and since the relative position between the radio-detectable elements of PH and the tracker T_PH is known by calibration, the transform matrix T_{Ref_ROI3→Ref_PH} between Ref_ROI3 and Ref_PH is determined (**figure 3** step c2). To this end, a single 2D image is necessary for the transform matrix T_{Ref_ROI3→Ref_PH}, any supplementary image will improve the redundancy and hence the accuracy of the transform matrix. One can note that in case of a 3D image, it will be the combination of for instance 180 images via a reconstruction algorithm.

At the time of the acquisition, the position of the tracker T_F with respect to T_PH is known, matrix transformation T_{Ref_F→Ref_PH} between Ref_F and Ref_PH is determined (**figure 3** step c3).

Transform matrix T_{Ref_ROI3→Ref_F} between Ref_ROI3 and Ref_F is determined (**figure 3** step c4) from T_{Ref_F→Ref_PH} and T_{Ref_ROI3→Ref_PH}.

Finally, any characteristic point located in the third region of interest ROI3 in Ref_ROI3 can be located with respect to Ref_F (**figure 3** steps c5 and c6).

In a preferred embodiment, the position (**figure 3** step c5) of any characteristic point in ROI3 is determined from the at least two projection images. To that end, from the positions of this characteristic point in each image, corresponding backprojection lines 35b, 36b are calculated, the position of the characteristic point in the reference system of the third region of interest being the intersection (or the closest point between the two backprojection lines) of these backprojection lines 35b, 36b. In a preferred embodiment, the characteristic point to be located relative to the third region of interest is the hip center. Therefore, for obtaining (**figure 3** step c5) the hip center, one possible set-up will be to acquire the 2D projection images with two images from the frontal plane taken at two different angles, allowing to detect on the image the hip ball and hence its center. In an alternative embodiment, for obtaining (**figure 3** step c5) the hip center, it is possible that an operator rotates the femur of the patient to find the center of rotation of the hip joint. That way, the hip center will be located in the reference system of the third region of interest and hence relatively to T_F. In other words, the hip center can be determined, with a functional method: a circumduction motion of the femur is performed around the hip. The navigation system records the 3D positions and orientations of the tracker T_F fixed to the femur during the motion. The center of rotation of the acquired data is computed and represents the hip center.

### Alternative implementation of steps a), b) and c)

The phantom PH can be realized according to various shapes: it can be an independent object of the set-up or included in a common object of a navigated robotic surgery. For instance, it can be included in the trackers T_F and T_T. In an embodiment, the trackers T_F and T_T may comprise radio-detectable elements and are visible in the image acquired at steps a1), b1), c1). In this embodiment, it is not necessary to use the calibration phantom PH. Therefore, the transform matrix between the reference system of the bone tracker and the reference system of the calibration phantom being a unitary matrix, the reference system of calibration phantom being equal to the reference system of the tracker bone.

### Figure 3 - step d: Location of any point in the ROI1 relative to any point in ROI2 and/or in ROI3

Once any characteristic point in the first region of interest is located relative to Ref_T and once any point in the second region of interest is located relative to Ref_T, it is therefore possible to locate any point in the first region of interest relative to any point in the second region interest.

Similarly, once any characteristic point in the first region of interest is located relative to Ref_F and once any point in the third region of interest is located relative to Ref_F, it is therefore possible to locate any point in the first region of interest relative to any point in the third region interest.

This location is particularly useful for obtaining alignment parameters around the knee joint and especially for planning a surgery (see below).

### Alignment parameters

In a preferred embodiment, a method, not forming part of the present invention, for obtaining alignment parameters comprises the steps a), b) and/or c) for locating the ankle center relative to the tracker T_T and/or for locating the hip center HC relative to the tracker T_F.

The ankle center, the hip center can be a characteristic point as such or can be the combination of several characteristic points. In that case, the ankle center is a characteristic element and requires the location of at least a first characteristic point and a second characteristic point. However, in the following we present the case where these centers are characteristic points as such but the method described here below also applies to characteristic element.

Therefore, it is then possible to determine (step d) the position of any characteristic point in the first region of interest ROI1 relative to tracker T_T and/or T_F in order to calculate alignment parameters of a femur bone and/or a tibia bone in said first region of interest (step e). For instance, it is possible to measure alignments between all three centers (hip center, knee center, angle center) in three planes (coronal, sagittal, frontal).

### High tibial osteotomy

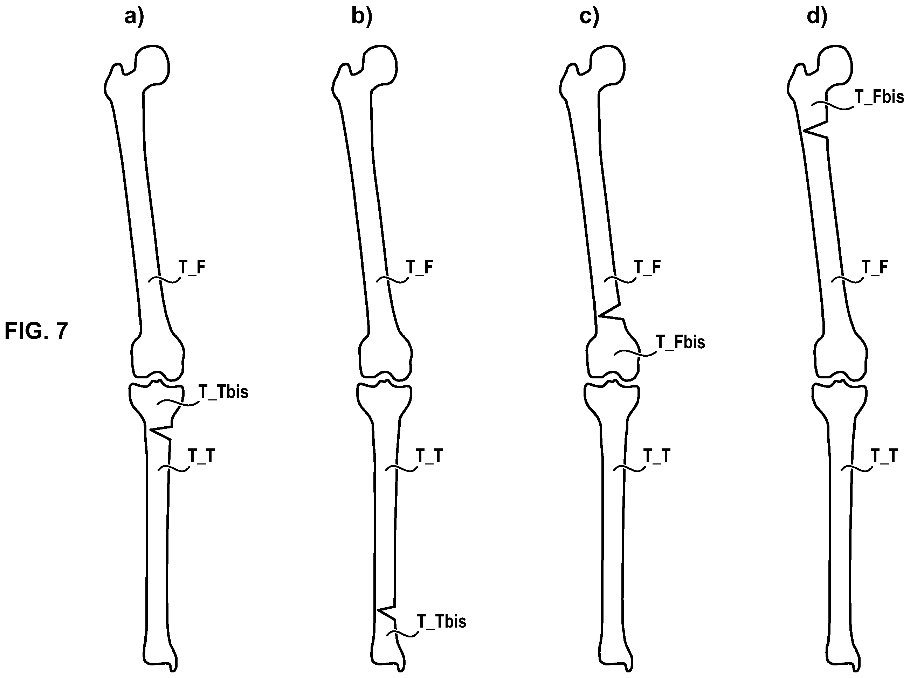

For the application to a high tibial osteotomy **(****figure 7a****),** open or closed wedge osteotomy, medial or lateral, a tracker T_Tbis similar to tracker T_T is fixed to the tibia bone so that so that trackers T_T and T_Tbis are on both sides of an osteotomy.

Tracker T_Tbis defines the reference system Ref_Tbis.

In a similar way to step a) from the image of the first region of interest, a transform matrix T_{Ref_ROI1→Ref_Tbis} between the reference system of the first region of interest and the reference system Ref_Tbis is determined. Therefore, it is possible to determine any characteristic point on the image of the first region of interest relative to tracker T_Tbis.

The relative positions of Ref_T and Ref_Tbis are monitored during the opening or closing of the osteotomy and until the fixation of the osteotomy to follow/ensure the execution of the optimal surgical plan **(****figure 3** step f).

### Distal tibial osteotomy

For the application to a distal tibial osteotomy **(****figure 7b****),** open or closed wedge osteotomy, medial or lateral, a tracker T_Tbis similar to tracker T_T is fixed to the tibia bone so that so that trackers T_T and T_Tbis are on both sides of an osteotomy.

Tracker T_Tbis defines a reference system Ref_Tbis.

In a similar way to step a) from the image of the first region of interest, a transform matrix T_{Ref_ROI1→Ref_Tbis} between the reference system of the first region of interest and the reference system Ref_Tbis is determined. Therefore, it is possible to determine any characteristic point in the image of the first region of interest relative to tracker T_Tbis.

The relative positions of Ref_T and Ref_Tbis are monitored during the opening or closing of the osteotomy and until the fixation of the osteotomy to follow/ensure the execution of the optimal surgical plan **(****figure 3** step f).

### Distal femoral osteotomy

For the application to a distal femoral osteotomy **(****figure 7c****),** open or closed wedge osteotomy, medial or lateral, a tracker T_Fbis similar to tracker T_F is fixed to the femur bone so that so that trackers T_F and T_Fbis are on both sides of an osteotomy.

Tracker T_Fbis defines a reference system Ref_Fbis.

In a similar way to step a) from the image of the first region of interest, a transform matrix T_{Ref_ROI1→Ref_Fbis} between the reference system of the first region of interest and the reference system Ref_Fbis is determined. Therefore, it is possible to determine any characteristic point on the image of the first region of interest relative to tracker T_Fbis.

The relative positions of Ref_F and Ref_Fbis are monitored during the opening or closing of the osteotomy and until the fixation of the osteotomy to follow/ensure the execution of the optimal surgical plan **(****figure 3** step f).

### High femoral osteotomy

For the application to a distal femoral osteotomy **(****figure 7d****),** open or closed wedge osteotomy, medial or lateral, a tracker T_Fbis similar to tracker T_F is fixed to the femur bone so that trackers T_F and T_Fbis are on both sides of an osteotomy.

Tracker T_Fbis defines a reference system Ref_Fbis.

In a similar way to step a), from the image of the first region of interest, a transform matrix T_{Ref_ROI1→Ref_Fbis} between the reference system of the first region of interest and the reference system Ref_Fbis is determined. Therefore, it is possible to determine any characteristic point in the image of the first region of interest relative to tracker T_Fbis.

The relative positions of Ref_F and Ref_Fbis are monitored during the opening or closing of the osteotomy and until the fixation of the osteotomy to follow/ensure the execution of the optimal surgical plan **(****figure 3** step f)).

### Taking into account of the kinematic

In an embodiment, the position of any characteristic point obtained consecutively to an application of external forces to the limb of the patient **(****figure 3** step f) can be located, relatively to other characteristic points in different positions of said limb, joint, when rotation or translations have been applied to the joint. One or several positions of the characteristic points and therefore the limb can be tracked and recorded during the application of external forces. These measurements are given as feedback on the display and are useful information for the understanding of the patient pathology.

### Knee arthroplasty

Knee arthroplasty is treatment option for patients with symptomatic osteoarthritis in at least one compartment of the knee. There are three compartments:
- Medial compartment (tibia + femur);
- Lateral compartment (tibia + femur);
- Patellofemoral compartment.

It is referred as Total knee arthroplasty (TKA) when at least the medial and lateral compartments are pathological or partial knee arthroplasty (PKA) when one compartment is pathological. A Unicompartmental Knee Arthroplasty (UKA) is realized when only one compartment (medial or lateral) is pathological.

For a TKA, bone cuts will be necessary at least on the tibia and the femur and could also be necessary on the patella. After the bones being cut, the tibia and femur prosthesis or implant will be fixed. Based on current common femoral prosthesis design, there are usually 3 bone cuts on the femur (anterior, distal and posterior) which are linked with 2 chamfer bone cuts, which make a total of 5 bone cuts on the femur. Based on current common tibial prosthesis design, on the tibia, there is one tibia cut. There may be a patella cut. The optimal femur prosthesis position and orientation is defined in Ref_F in order to optimize at least one of the following criteria:
1. Angle between the Femoral component of the Knee Prothesis (KPF) axis passing through its center KPF and its longitudinal axis and the segment KPF-HC close to zero degrees
2. Anterior surface of the prosthesis as flush as possible to the anterior surface of the bone detected on the 3D image
3. Axial orientation of the prosthesis parallel to a line of two posterior or epicondyle landmarks detected on the 3D image
4. No overlap of the prosthesis outside the bone detected on the 3D image in the medio-lateral direction
5. Height of the prosthesis respecting the initial joint line
6. Minimal positive gaps between the femur and the tibia for a given flexion extension of the knee as simulated using kinematic data

The optimal tibia prosthesis position and orientation is defined in Ref_T in order to optimize at least one of the following criteria:
1. Angle in the frontal plane between the Tibial component of the Knee Prothesis (KPT) axis passing through its center KPT and its longitudinal axis and the segment KPT-AC close to zero degrees
2. No overlap of the prosthesis outside the bone detected on the 3D image in the medio-lateral and antero-posterior directions
3. Height of the prosthesis respecting the initial joint line
4. Tibial slope set to a predefined value with respect to the initial slope detected on the 3D image
5. Tibial rotation set to a predefined value with respect to a sagittal plane defined in the 3D image

For a UKA, bone cuts will be necessary on the tibia and the femur. After the bones being cut, the tibia and femur prosthesis or implant will be fixed. On the femur, there are usually two bone cuts (distal and posterior) or a milling cut which are linked with 1 chamfer bone cut (anterior chamfer), which make a total of 3 bone cuts on the femur. On the tibia, there are two perpendicular tibia cuts. The optimal femur prosthesis position and orientation is defined in Ref_F in order to optimize at least one of the following criteria:
1. Angle between the Femoral component of the Knee Prothesis (KPF) axis passing through its center KPF and its longitudinal axis and the segment KPF-HC close to zero degrees
2. Anterior surface of the prosthesis as flush as possible to the anterior surface of the bone detected on the 3D image
3. Axial orientation of the prosthesis parallel to a line of one posterior or epicondyle landmarks detected on the 3D image
4. No overlap of the prosthesis outside the bone detected on the 3D image in the medio-lateral direction
5. Height of the prosthesis respecting the initial joint line
6. Minimal positive gaps between the femur and the tibia for a given flexion extension of the knee as simulated using kinematic data

The optimal tibia prosthesis position and orientation is defined in Ref_T in order to optimize at least one of the following criteria:
1. Angle in the frontal plane between the Tibial component of the Knee Prothesis (KPT) axis passing through its center KPT and its longitudinal axis and the segment KPT-AC close to zero degrees
2. No overlap of the prosthesis outside the bone detected on the 3D image in the medio-lateral and antero-posterior directions
3. Height of the prosthesis respecting the initial joint line
4. Tibial slope set to a predefined value with respect to the initial slope detected on the 3D image
5. Tibial rotation set to a predefined value with respect to a sagittal plane defined in the 3D image

Additionally, for all knee arthroplasty, the optimal femur and tibia prosthesis respective position and orientation and as defined in Ref_T and Ref_F may be linked so as to respect an angle HC-KC-AC below a certain value, such as below 3° varum or valgum depending of the original patient deformity.

There exists other technics for performing TKA or PKA that may require specific tibia, femur or patella bone cuts. For instance, Cruciate Retaining TKA, Bicruciate Retaining TKA, and other specific methods. For these specific TKAs, the optimal femur prosthesis and tibia prothesis positions and orientations are defined in Ref_F and Ref_T respectively in order to optimize specific criteria which may (or not) be the same as previously described for a TKA.

The system is thus well adapted to perform femoral or tibial osteotomy or double level osteotomy procedure wherein:
a. The medical system calculates, from the clinical goal correction given by the practitioner and the images of the regions of interest acquired by the medical system, the final position of the tibia bone, femur bone and the first or second end of the bone where the osteotomy occurs.
b. The determination of characteristic points allows to plan optimal osteotomy cuts in Ref_F and/or Ref_T, wherein said optimal cuts are performed using robotics or surgical navigation assistance.
c. The medical system gives a feedback to the practitioner, via or not the display, to compare the final positions of the bones calculated by the medical system and the actual position measured in real time via the medical system.

### Legends of the figures

| | | |
|---|---|---|
| | P | Pelvis |
| | HC | Hip Center |
| | F | Femur |
| | K | Knee Joint |
| Figure 1 | KC | Knee Center |
| | T | Tibia |
| | AC | Ankle Center |
| | FO | Foot |
| | HC-K | Mechanical axis of the femur |
| | KC-AC | Mechanical axis of the tibia |

| | | | |
|---|---|---|---|
| | 100: Medical system | 101 | Second processing unit |
| | | 102 | Storage unit |
| Figure 2 | | 103 | Displays |
| | 200: Operating table | | |
| | 300: Patient | | |
| | | 401 | X-ray source |
| | 400: imaging device | 402 | X-ray detector |
| | | 403 | C-arm |
| | | 404 | First processing unit |
| | 500: navigation system | 501 | (optional) External localizer |
| | | 502 | Tracker of the tibia |
| | | 503 | Tracker of the femur |
| | | 504 | Tracker of the calibration phantom |

| | | | |
|---|---|---|---|
| | a) | a1) | X-ray images of the ROI1 (2D or 3D), the relative positions of both tracker F and T with the tracker of the calibration phantom is recorded at the time of each image acquisition: a3) and a5) |
| | | a2) | Matrix transformation between Ref_ROI1 and Ref_PH |
| | | a3) | Matrix transformation between Ref_F and Ref_PH at the time of one image acquisition |
| | | a4) | Matrix transformation between Ref_T and Ref_PH at the time of one image acquisition |
| | | a5) | Matrix transformation between Ref_ROI1 and Ref_F |
| | | a6) | Matrix transformation between Ref_ROI1 and Ref_T |
| | | a7) | Point in ROI1 |
| Figure 3 | | a8) | Point in ROI1 expressed in Ref_F |
| | | a9) | Point in ROI1 expressed in Ref_T |
| | | b1) | X-ray images of the ROI2 (2D or 3D), the relative positions of tracker T with the tracker of the calibration phantom is recorded at the time of each image acquisition: b3) |
| | | b2) | Matrix transformation between Ref_ROI2 and Ref_PH |
| | b) | b3) | Matrix transformation between Ref_T and Ref_PH at the time of one image acquisition |
| | | b4) | Matrix transformation between Ref_ROI2 and Ref_T |
| | | b5) | Point in ROI2 |
| | | b6) | Point in ROI2 expressed in Ref_T |
| | | c1) | X-ray images of the ROI3 (2D or 3D), the relative positions of tracker F with the tracker of the calibration phantom is recorded at the time of each image acquisition: c3) |
| | | c2) | Matrix transformation between Ref_ROI3 and Ref_PH |
| | c) | c3) | Matrix transformation between Ref_F and Ref_PH at the time of one image acquisition |
| | | c4) | Matrix transformation between Ref_ROI3 and Ref_F |
| | | c5) | Point in ROI3 |
| | | c6) | Point in ROI3 expressed in Ref_F |
| | d) | | Any point in the ROI1 can be located relative to any points in ROI2 and/or in ROI3 |
| | e) | | Alignment parameters |
| | f) | | Real time tracking |

| | | |
|---|---|---|
| | 10 | First region of interest |
| | 11 | Knee joint |
| | 12 | X-ray image |
| | 13 | Representation of the knee joint on the image |
| Figure 4 | 14 | different positions of the X-ray source |
| | 15 | Frame of reference Ref_ROI1 of the imaging device defined in this first region of interest |
| | 16 | tracker T_F |
| | 17 | tracker T_T |
| | 18 | calibration phantom PH |

| | | |
|---|---|---|
| | 20a | Second region of interest |
| | 20b | Frame of reference Ref_ROI2 of the imaging device defined in this second region of interest |
| | 21 | X-ray projection image |
| | 22 | X-ray projection image |
| | 23 | Position of the X-ray source for the image acquisition 22 |
| | 24 | Position of the X-ray source for the image acquisition 21 |
| Figure 5 | 25a | Ankle center (or any relevant characteristic point of the ankle joint) visible on projection images 21 |
| | 25b | Corresponding backprojection lines of 25a |
| | 26a | Ankle center (or any relevant characteristic point of the ankle joint) visible on projection images 22 |
| | 26b | Corresponding backprojection lines of 26a |
| | 27 | ankle center |
| | 52 | calibration phantom PH |
| | 51 | tracker T_T |

| | | |
|---|---|---|
| | 30a | Third region of interest |
| | 30b | Frame of reference Ref_ROI3 of the imaging device defined in this third region of interest |
| | 31 | X-ray projection image |
| | 32 | X-ray projection image |
| | 33 | Position of the X-ray source for the image acquisition 32 |
| Figure 6 | 34 | Position of the X-ray source for the image acquisition 31 |
| | 35a | Hip center (or any relevant characteristic point of the hip joint) visible on projection images 31 |
| | 35b | Corresponding backprojection lines of 35a |
| | 36a | Hip center(or any relevant characteristic point of the hip joint) visible on projection images 32 |
| | 36b | Corresponding backprojection lines of 36a |
| | 37 | Hip center |
| | 52 | calibration phantom PH |
| | 50 | tracker T_F |

## Claims

1. Medical system for locating at least one characteristic point in a region of a joint having a joint center (AC, HC, KC), the joint center (AC, HC, KC) being an end of a bone (T, F), a bone tracker (T_F, T_T) has been fixed to said bone and defines a reference system (Ref_T, Ref_F), the medical system comprising a processor configured for:
- acquiring (a1, b1, c1) at least one image from an imaging device of a region of interest of a joint (ROI1, ROI2, ROI3) comprising or near the joint center, a calibration phantom PH with a tracker T_PH being visible in the image, said calibration phantom PH being in the field of view of the imaging device during the acquisition of the image, the relative position between radio-detectable elements of PH and the tracker T_PH being known, the tracker T_PH being located in a reference system Ref_PH, the position of the bone tracker (T_F, T_T) with respect to T_PH being known at the time of acquisition;
- determining (a2, b2, c2) a transform matrix between the reference system of the calibration phantom PH and the reference system of the region of interest from the position of the calibration phantom PH in the acquired image, the calibration phantom being located in said region of interest and in the reference system of the region of interest;
- determining (a5, a6, b4, c4) from a transform matrix between the reference system of the bone tracker and the reference system (a3, b3, c3) of the calibration phantom and from the transform matrix between the reference system of the calibration phantom and the reference system of the region of interest (a2, b2, c2), the transform matrix between the reference system of the region of interest and the reference system of the bone tracker;
- determining (a7, b5, c5) a position of each of the at least one characteristic point in the reference system of the region of interest of the joint;
- determining (a8, b6, c6) the location of each of the said at least one characteristic point in the region of interest relatively to reference system of the bone tracker (T_F, T_T) from the transform matrix between the reference system of the region of interest and the reference system of the bone tracker and from the position of said characteristic point in the reference system of the region of interest of the joint.

2. The medical system according to claim 1, wherein the processor is configured for acquiring at least two 2D projection images, and wherein for determining (a7, b5, c5) the position of the characteristic point in the region of interest the processor is configured for calculating at least two backprojection lines corresponding to the position of the characteristic point in each projection image, the position of the characteristic point in the reference system of the region of interest, being the intersection between these backprojection lines.

3. The medical system according to claim 2, wherein the characteristic point is the ankle center (AC), and wherein the 2D projection images are orthogonal acquisitions, preferably frontal and sagittal to locate the characteristic points on the talus and the distal tibia.

4. The medical system according to claim 2, wherein the characteristic point is the ankle center (AC), wherein the 2D projection images correspond to acquisition with several orientations, preferably +15° and - 15° from the frontal orientation to detect malleoli of the tibia and fibula.

5. The medical system according to claim 2, wherein the characteristic point is the hip center (HC), the 2D projection images (a1, b1, c1) correspond to acquisitions with several orientations, preferably to +30° and -30° from the frontal orientation to detect the center of the hip ball.

6. The medical system according to any one of the preceding claims, wherein at least one 3D image is acquired.

7. The medical system according to any one of the preceding claims, wherein radio-detectable elements of the calibration phantom are part of the bone tracker and are in the field of view and visible in the image, said transform matrix between the reference system of the bone tracker and the reference system of the calibration phantom being a unitary matrix, the reference system of calibration phantom being equal to the reference system of the tracker bone.

8. The medical system according to any one of the preceding claims, wherein a single characteristic point is located or a characteristic element is located which is a combination of at least a first and second characteristic points located, the single characteristic point or the characteristic element being preferably the ankle center (AC), the hip center (HC) or the knee center (KC).

9. The medical system according to any one of the preceding claims, wherein the processor is further configured for determining alignment parameters of a tibia bone, a first end of the tibia bone being an ankle joint, a second end of tibia bone being a knee joint, a tracker T_T has been fixed to the tibia bone and defines a reference system Ref_T by:
- locating at least one characteristic point in a first region of interest (ROI1) comprising the knee joint relatively to the reference system Ref_T;
- locating at least one characteristic point in a second region of interest (ROI2) comprising the ankle joint relatively to the reference system Ref_T;
- calculating at least one alignment parameter (e) from the location of said characteristic points located relatively to Ref_T in the first and/or second of region of interest(s).

10. The medical system as claimed in the preceding claim, wherein a tracker T_Tbis has been fixed to the tibia, so that trackers T_T and T_Tbis are on both sides of an osteotomy, the tracker T_Tbis defining a reference system Ref_Tbis. said processor further configured for locating at least one characteristic point in the first region of interest (ROI1) relative to Ref_Tbis and/or comprising locating at least one characteristic point in the second of interest relative to Ref_Tbis.

11. The medical system according to any one of claims 1 to 8, wherein the processor is further configured for determining alignment parameters of a femur bone, a first end of the femur bone being a hip joint, a second end of femur bone being a knee joint, a tracker T_F has been fixed to the femur bone and defines a reference system Ref_F byr:
- locating at least one characteristic point in a first region of interest (ROI) comprising the knee joint relatively to the reference system Ref_F;
- locating at least one characteristic point such as the hip center in the third region (ROI3) of interest comprising the hip center relatively to the reference system Ref_F;
- calculating at least one alignment parameter from the location of said characteristic points located relatively to Ref_F in the first and/or second region of interest(s) (ROI1, ROI2).

12. The medical system as claimed in the preceding claim, a tracker T_Fbis has been fixed to the femur, so that trackers T_F and T_Fbis are on both sides of an osteotomy, tracker T_Fbis defining a reference system Ref_Fbis, the medical system being configured for locating at least one characteristic point in the first region of interest (ROI1) relative to Ref_Fbis and/or comprising locating at least one characteristic point in the third region of interest (ROI3) relative to Ref_Fbis.

13. The medical system as claimed in claims 11 to 12, wherein the location of any characteristic point in the first region of interest (ROI1) comprising the knee joint is obtained consecutively to an application of external forces to the limb of the patient so that the localization of characteristic points can be tracked in corresponding different positions of said joint, rotation or translations have been applied to the joint.

14. The medical system as claimed in claims 9 to 13, wherein the processor is further configured for planning an optimal surgery around a knee joint of a patient, by determining alignment parameters (e) of a femur bone and a tibia bone, a surgery being planned based on these parameters in order to be executed manually or by a robot.

15. The medical system as claimed in claim 14, comprising a device for implementing the planned surgical procedure.

## Patentansprüche

1. Medizinisches System zum Lokalisieren von mindestens einem charakteristischen Punkt in einer Region eines Gelenks, das ein Gelenkzentrum (AC, HC, KC) aufweist, wobei das Gelenkzentrum (AC, HC, KC) ein Ende eines Knochens (T, F) ist, wobei ein Knochenverfolger (T_F, T_T) an dem Knochen befestigt wurde und ein Referenzsystem (Ref_T, Ref_F) definiert, wobei das medizinische System einen Prozessor umfasst, der zu Folgendem ausgelegt ist:
- Erfassen (a1, b1, c1) von mindestens einem Bild von einer Bildgebungsvorrichtung einer Region von Interesse eines Gelenks (ROI1, ROI2, ROI3), die das Gelenkzentrum umfasst oder demselben nahe ist, wobei im Bild ein Kalibrierungsphantom PH mit einem Verfolger T_PH sichtbar ist, wobei sich das Kalibrierungsphantom PH während der Erfassung des Bildes im Sichtfeld der Bildgebungsvorrichtung befindet, wobei die relative Position zwischen mittels Funk detektierbaren Elementen von PH und dem Verfolger T_PH bekannt ist, wobei der Verfolger T_PH sich in einem Referenzsystem Ref_PH befindet, wobei die Position des Knochenverfolgers (T_F, T_T) mit Bezug auf T_PH zur Zeit der Erfassung bekannt ist
- Bestimmen (a2, b2, c2) einer Transformationsmatrix zwischen dem Referenzsystem des Kalibrierungsphantoms PH und dem Referenzsystem der Region von Interesse anhand der Position des Kalibrierungsphantoms PH im erfassten Bild, wobei sich das Kalibrierungsphantom in der Region von Interesse und im Referenzsystem der Region von Interesse befindet;
- Bestimmen (a5, a6, b4, c4) der Transformationsmatrix zwischen dem Referenzsystem der Region von Interesse und dem Referenzsystem des Knochenverfolgers anhand einer Transformationsmatrix zwischen dem Referenzsystem des Knochenverfolgers und dem Referenzsystem (a3, b3, c3) des Kalibrierungsphantoms sowie anhand der Transformationsmatrix zwischen dem Referenzsystem des Kalibrierungsphantoms und dem Referenzsystem der Region von Interesse (a2, b2, c2);
- Bestimmen (a7, b5, c5) einer Position von jedem des mindestens einen charakteristischen Punktes im Referenzsystem der Region von Interesse des Gelenks;
- Bestimmen (a8, b6, c6) des Ortes von jedem des mindestens einen charakteristischen Punktes in der Region von Interesse relativ zum Referenzsystem des Knochenverfolgers (T_F, T_T) anhand der Transformationsmatrix zwischen dem Referenzsystem der Region von Interesse und dem Referenzsystem des Knochenverfolgers sowie anhand der Position des charakteristischen Punktes im Referenzsystem der Region von Interesse des Gelenks.

2. Medizinisches System nach Anspruch 1, wobei der Prozessor zum Erfassen von mindestens zwei 2D-Projektionsbildern ausgelegt ist, und wobei zum Bestimmen (a7, b5, c5) der Position des charakteristischen Punktes in der Region von Interesse der Prozessor zum Berechnen von mindestens zwei Rückprojektionslinien ausgelegt ist, die der Position des charakteristischen Punktes in jedem Projektionsbild entsprechen, wobei die Position des charakteristischen Punktes im Referenzsystem der Region von Interesse der Schnittpunkt zwischen diesen Rückprojektionslinien ist.

3. Medizinisches System nach Anspruch 2, wobei der charakteristische Punkt das Knöchelzentrum (AC) ist, und wobei die 2D-Projektionsbilder orthogonale Erfassungen sind, vorzugsweise frontal und sagittal, um die charakteristischen Punkte auf dem Talus und der distalen Tibia zu lokalisieren.

4. Medizinisches System nach Anspruch 2, wobei der charakteristische Punkt das Knöchelzentrum (AC) ist, wobei die 2D-Projektionsbilder einer Erfassung mit mehreren Orientierungen entsprechen, vorzugsweise +15° und -15° aus der frontalen Orientierung, um Malleoli der Tibia und der Fibula zu detektieren.

5. Medizinisches System nach Anspruch 2, wobei der charakteristische Punkt das Hüftzentrum (HC) ist, die 2D-Projektionsbilder (a1, b1, c1) Erfassungen mit mehreren Orientierungen entsprechen, vorzugsweise +30 ° und -30° von der frontalen Orientierung, um das Zentrum des Hüftkopfes zu detektieren.

6. Medizinisches System nach einem der vorhergehenden Ansprüche, wobei mindestens ein 3D-Bild erfasst wird.

7. Medizinisches System nach einem der vorhergehenden Ansprüche, wobei die mittels Funk detektierbaren Elemente des Kalibrierungsphantoms Teil des Knochenverfolgers sind und sich im Sichtfeld befinden und im Bild sichtbar sind, wobei die Transformationsmatrix zwischen dem Referenzsystem des Knochenverfolgers und dem Referenzsystem des Kalibrierungsphantoms eine unitäre Matrix ist, wobei das Referenzsystem des Kalibrierungsphantoms gleich dem Referenzsystem des Knochenverfolgers ist.

8. Medizinisches System nach einem der vorhergehenden Ansprüche, wobei ein einzelner charakteristischer Punkt oder ein charakteristisches Element lokalisiert wird, das eine Kombination von mindestens einem lokalisierten ersten und zweiten charakteristischen Punkt ist, wobei der einzelne charakteristische Punkt oder das charakteristische Element vorzugsweise das Knöchelzentrum (AC), das Hüftzentrum (HC) oder das Kniezentrum (KC) ist.

9. Medizinisches System nach einem der vorhergehenden Ansprüche, wobei der Prozessor ferner zum Bestimmen von Ausrichtungsparametern eines Tibiaknochens ausgelegt ist, wobei ein erstes Ende des Tibiaknochens ein Fußgelenk ist, wobei ein zweites Ende des Tibiaknochens ein Kniegelenk ist, wobei ein Verfolger T_T am Tibiaknochen befestigt wurde und ein Referenzsystem Ref_T durch Folgendes definiert:
- Lokalisieren von mindestens einem charakteristischen Punkt in einer ersten Region von Interesse (ROI1), die das Kniegelenk relativ zum Referenzsystem Ref_T umfasst;
- Lokalisieren von mindestens einem charakteristischen Punkt in einer zweiten Region von Interesse (ROI2), die das Fußgelenk relativ zum Referenzsystem Ref_T umfasst;
- Berechnen von mindestens einem Ausrichtungsparameter (e) anhand des Ortes der lokalisierten charakteristischen Punkte relativ zu Ref_T in der ersten und/oder der zweiten Region von Interesse.

10. Medizinisches System wie im vorhergehenden Anspruch beansprucht, wobei ein Verfolger T_Tbis an der Tibia befestigt wurde, derart, dass sich Verfolger T_T und T_Tbis auf beiden Seiten einer Osteotomie befinden, wobei der Verfolger T_Tbis eine Referenzsystem Ref_Tbis definiert, wobei der Prozessor ferner zum Lokalisieren von mindestens einem charakteristischen Punkt in der ersten Region von Interesse (ROI1) relativ zu Ref_Tbis ausgelegt ist und/oder das Lokalisieren von mindestens einem charakteristischen Punkt in der zweiten von Interesse relativ zu Ref_Tbis umfasst.

11. Medizinisches System nach einem der Ansprüche 1 bis 8, wobei der Prozessor ferner zum Bestimmen von Ausrichtungsparametern eines Femurknochens ausgelegt ist, wobei ein erstes Ende des Femurknochens ein Hüftgelenk ist, wobei ein zweites Ende des Femurknochens ein Kniegelenk ist, wobei ein Verfolger T_F am Femurknochen befestigt wurde und ein Referenzsystem Ref_F durch Folgendes definiert:
- Lokalisieren von mindestens einem charakteristischen Punkt in einer ersten Region von Interesse (ROI), die das Kniegelenk relativ zum Referenzsystem Ref_F umfasst;
- Lokalisieren von mindestens einem charakteristischen Punkt, wie etwa des Hüftzentrums, in der dritten Region (ROI3) von Interesse, die das Hüftzentrum relativ zum Referenzsystem Ref_F umfasst;
- Berechnen von mindestens einem Ausrichtungsparameter anhand des Ortes der lokalisierten charakteristischen Punkte relativ zu Ref_F in der ersten und/oder der zweiten Region von Interesse (ROI1, ROI2).

12. Medizinisches System wie im vorhergehenden Anspruch beansprucht, wobei ein Verfolger T_Fbis am Femur befestigt wurde, derart, dass sich Verfolger T_F und T_Fbis auf beiden Seiten einer Osteotomie befinden, wobei Verfolger T_Fbis ein Referenzsystem Ref_Fbis definiert, wobei das medizinische System zum Lokalisieren von mindestens einem charakteristischen Punkt in der ersten Region von Interesse (ROI1) relativ zu Ref_Fbis ausgelegt ist und/oder das Lokalisieren von mindestens einem charakteristischen Punkt in der dritten Region von Interesse (ROI3) relativ zu Ref_Fbis umfasst.

13. Medizinisches System wie in den Ansprüchen 11 bis 12 beansprucht, wobei der Ort eines charakteristischen Punktes in der ersten Region von Interesse (ROI1), die das Kniegelenk umfasst, nach einer Anwendung von externen Kräften auf das Gliedmaß des Patienten erhalten wird, derart, dass die Lokalisierung von charakteristischen Punkten in entsprechenden verschiedenen Positionen des Gelenks verfolgt werden kann, wobei eine Rotation oder Translationen auf das Gliedmaß angewandt wurden.

14. Medizinisches System wie in den Ansprüchen 9 bis 13 beansprucht, wobei der Prozessor ferner zum Planen einer optimalen Chirurgie um ein Kniegelenk eines Patienten durch Bestimmen von Ausrichtungsparametern (e) eines Femurknochens oder eines Tibiaknochens ausgelegt ist, wobei eine Chirurgie auf Basis dieser Parameter geplant wird, um manuell oder durch einen Roboter ausgeführt zu werden.

15. Medizinisches System wie in Anspruch 14 beansprucht, das eine Vorrichtung zum Implementieren der geplanten chirurgischen Prozedur umfasst.

## Revendications

1. Système médical de localisation d'au moins un point caractéristique dans une région d'une articulation ayant un centre d'articulation (AC, HC, KC), le centre d'articulation (AC, HC, KC) étant une extrémité d'un os (T, F), un traqueur osseux (T_F, T_T) ayant été fixé audit os et définissant un système de référence (Ref_T, Ref_F), le système médical comprenant un processeur configuré pour :
- acquérir (a1, b1, c1) au moins une image à partir d'un dispositif d'imagerie d'une région d'intérêt d'une articulation (ROI1, ROI2, ROI3) comprenant le centre de l'articulation ou étant située à proximité de celui-ci, un fantôme d'étalonnage PH muni d'un traqueur T_PH étant visible sur l'image, ledit fantôme d'étalonnage PH se trouvant dans le champ de vision du dispositif d'imagerie pendant l'acquisition de l'image, la position relative entre les éléments radiodétectables PH et le traqueur T_PH étant connue, le traqueur T_PH étant situé dans un système de référence Ref_PH, la position du traqueur osseux (T_F, T_T) par rapport à T_PH étant connue au moment de l'acquisition ;
- déterminer (a2, b2, c2) une matrice de transformation entre le système de référence du fantôme d'étalonnage PH et le système de référence de la région d'intérêt à partir de la position du fantôme d'étalonnage PH dans l'image acquise, le fantôme d'étalonnage étant situé dans ladite région d'intérêt et dans le système de référence de la région d'intérêt ;
- déterminer (a5, a6, b₄, c₄) à partir d'une matrice de transformation entre le système de référence du suiveur osseux et le système de référence (a₃, b₃, c₃) du fantôme d'étalonnage, et à partir de la matrice de transformation entre le système de référence du fantôme d'étalonnage et le système de référence de la région d'intérêt (a₂, b₂, c₂), la matrice de transformation entre le système de référence de la région d'intérêt et le système de référence du traqueur osseux ;
- déterminer (a7, b5, c5) une position de chacun des au moins un point caractéristique dans le système de référence de la région d'intérêt de l'articulation ;
- déterminer (a8, b6, c6) l'emplacement de chacun desdits au moins un point caractéristique dans la région d'intérêt par rapport au système de référence du traqueur osseux (T_F, T_T) à partir de la matrice de transformation entre le système de référence de la région d'intérêt et le système de référence du traqueur osseux (T_F, T_T) et à partir de la position dudit point caractéristique dans le système de référence de la région d'intérêt de l'articulation.

2. Système médical selon la revendication 1, dans lequel le processeur est configuré pour acquérir au moins deux images de projection 2D, et dans lequel, pour déterminer (a7, b5, c5) la position du point caractéristique dans la région d'intérêt, le processeur est configuré pour calculer au moins deux lignes de rétroprojection correspondant à la position du point caractéristique dans chaque image de projection, la position du point caractéristique dans le système de référence de la région d'intérêt étant le point d'intersection entre ces lignes de rétroprojection.

3. Système médical selon la revendication 2, dans lequel le point caractéristique est le centre de la cheville (AC), et dans lequel les images de projection 2D sont des acquisitions orthogonales, de préférence frontales et sagittales, afin de localiser les points caractéristiques sur l'astragale et le tibia distal.

4. Système médical selon la revendication 2, dans lequel le point caractéristique est le centre de la cheville (AC), et dans lequel les images de projection 2D correspondent à des acquisitions selon plusieurs orientations, de préférence à +15° et -15° par rapport à l'orientation frontale, afin de détecter les malléoles du tibia et du péroné.

5. Système médical selon la revendication 2, dans lequel le point caractéristique est le centre de la hanche (HC), les images de projection en 2D (a1, b1, c1) correspondent à des acquisitions selon plusieurs orientations, de préférence à +30° et -30° par rapport à l'orientation frontale, afin de détecter le centre de la tête fémorale.

6. Système médical selon l'une quelconque des revendications précédentes, dans lequel au moins une image 3D est acquise.

7. Système médical selon l'une quelconque des revendications précédentes, dans lequel des éléments radiodétectables du fantôme d'étalonnage font partie du traqueur osseux et se trouvent dans le champ de vision et sont visibles sur l'image, ladite matrice de transformation entre le système de traqueur osseux et le système de référence du fantôme d'étalonnage étant une matrice unitaire, le système de référence du fantôme d'étalonnage étant identique au système de référence du traqueur osseux.

8. Système médical selon l'une quelconque des revendications précédentes, dans lequel un point caractéristique unique est localisé ou un élément caractéristique est localisé, lequel est une combinaison d'au moins un premier et un deuxième points caractéristiques localisés, le point caractéristique unique ou l'élément caractéristique étant de préférence le centre de la cheville (AC), le centre de la hanche (HC) ou le centre du genou (KC).

9. Système médical selon l'une quelconque des revendications précédentes, dans lequel le processeur est en outre configuré pour déterminer les paramètres d'alignement d'un tibia, une première extrémité du tibia étant une articulation de la cheville, une seconde extrémité du tibia étant une articulation du genou, un traceur T_T ayant été fixé au tibia et définissant un système de référence Ref_T par :
- localiser au moins un point caractéristique dans une première région d'intérêt (ROI1) comprenant l'articulation du genou par rapport au système de référence Ref_T ;
- localiser au moins un point caractéristique dans une deuxième région d'intérêt (ROI2) comprenant l'articulation de la cheville par rapport au système de référence Ref_T ;
- calculer au moins un paramètre d'alignement (e) à partir de la position desdits points caractéristiques localisés par rapport à Ref_T dans la première et/ou la deuxième région d'intérêt.

10. Système médical selon la revendication précédente, dans lequel un capteur T_Tbis a été fixé au tibia, de sorte que les capteurs T_T et T_Tbis se trouvent de part et d'autre d'une ostéotomie, le traceur T_Tbis définissant un système de référence Ref_Tbis, ledit processeur étant en outre configuré pour localiser au moins un point caractéristique dans la première région d'intérêt (ROI1) par rapport à Ref_Tbis et/ou comprenant la localisation d'au moins un point caractéristique dans la deuxième région d'intérêt par rapport à Ref_Tbis.

11. Système médical selon l'une quelconque des revendications 1 à 8, dans lequel le processeur est en outre configuré pour déterminer des paramètres d'alignement d'un fémur, une première extrémité du fémur étant une articulation de la hanche, une seconde extrémité du fémur étant une articulation du genou, un capteur T_F ayant été fixé au fémur et définissant un système de référence Ref_F, en :
- localiser au moins un point caractéristique dans une première région d'intérêt (ROI) comprenant l'articulation du genou par rapport au système de référence Ref_F ;
- localiser au moins un point caractéristique, tel que le centre de la hanche, dans une troisième région d'intérêt (ROI3) comprenant le centre de la hanche par rapport au système de référence Ref_F ;
- calculer au moins un paramètre d'alignement à partir de la localisation desdits points caractéristiques situés par rapport à Ref_F dans la ou les première(s) et/ou deuxième(s) régions d'intérêt (ROI1, ROI2).

12. Système médical selon la revendication précédente, dans lequel un traqueur T_Fbis a été fixé au fémur, de sorte que les traqueurs T_F et T_Fbis se trouvent de part et d'autre d'une ostéotomie, le traqueur T_Fbis définissant un système de référence Ref_Fbis, le système médical étant configuré pour localiser au moins un point caractéristique dans la première région d'intérêt (ROI1) par rapport à Ref_Fbis et/ou comprenant la localisation d'au moins un point caractéristique dans la troisième région d'intérêt (ROI3) par rapport à Ref_Fbis.

13. Système médical selon les revendications 11 à 12, dans lequel la localisation de tout point caractéristique dans la première région d'intérêt (ROI1) comprenant l'articulation du genou est obtenue consécutivement à l'application de forces externes sur le membre du patient, de sorte que la localisation des points caractéristiques puisse être suivie dans différentes positions correspondantes de ladite articulation, lorsque des rotations ou des translations ont été appliquées à l'articulation.

14. Système médical selon les revendications 9 à 13, dans lequel le processeur est en outre configuré pour planifier une intervention chirurgicale optimale autour d'une articulation du genou d'un patient, en déterminant des paramètres d'alignement (e) d'un fémur et d'un tibia, une intervention chirurgicale étant planifiée sur la base de ces paramètres afin d'être exécutée manuellement ou par un robot.

15. Système médical selon la revendication 14, comprenant un dispositif destiné à mettre en œuvre l'intervention chirurgicale planifiée.
